# EUROPÄISCHE PATENTSCHRIFT

(11) **EP 1 296 756 B1**
(45) Veröffentlichungstag und Bekanntmachung des Hinweises auf die Patenterteilung: **27.09.2006**
(21) Anmeldenummer: 01927823.3
(22) Anmeldetag: 28.03.2001
(51) Int. Cl.: B01J 19/00, C07K 1/04, C07H 21/00

(54) **VERFAHREN UND VORRICHTUNG ZUR UNTERSUCHUNG DER MOLEKULAREN WECHSELWIRKUNGEN VON LÖSLICHEN ODER SUSPENDIERBAREN WIRKSTOFFEN MIT FESTPHASEN-GEBUNDENEN PEPTIDISCHEN ODER PEPTOIDEN ZIELMOLEKÜLEN DURCH KAPILLARPLATTEN MIT GROSSER INNERER OBERFLÄCHE**
METHOD AND DEVICE FOR INVESTIGATING THE MOLECULAR INTERACTION OF SOLUBLE OR SUSPENDABLE ACTIVE SUBSTANCES WITH SOLID-PHASE BONDED PEPTIDIC OR PEPTOID TARGET MOLECULES BY MEANS OF CAPILLARY PLATES HAVING A LARGE INNER SURFACE
PROCEDE ET DISPOSITIF POUR ETUDIER LES INTERACTIONS MOLECULAIRES DE PRINCIPES ACTIFS, SOLIDES OU POUVANT ETRE MIS EN SUSPENSION, AVEC DES MOLECULES CIBLES PEPTIDIQUES OU PEPTOIDES LIEES A UNE PHASE SOLIDE, PAR L'INTERMEDIAIRE DE PLAQUES DE CAPILLAIRES PRESENTANT UNE GRANDE SURFACE INTERIEURE

(30) Priorität: 28.03.2000 DE 10015391; 28.03.2000 DE 20005738 U
(43) Veröffentlichungstag der Anmeldung: 02.04.2003
(73) Patentinhaber: Laser- und Medizin-Technologie GGmbH Berlin, 14195 Berlin (DE)
(72) Erfinder: FREY, Andreas, 23816 Gross Niendorf (DE); HELFMANN, Jürgen, 14532 Kleinmachnow (DE); SCHMIDT, Marcus, Alexander, 48329 Havixbeck (DE); MÜLLER, Gerhard, 14129 Berlin (DE)
(74) Vertreter: Eisenführ, Speiser & Partner
(86) Internationale Anmeldenummer: PCT/EP2001/003530
(87) Internationale Veröffentlichungsnummer: WO 2001/072412

(56) Entgegenhaltungen:
- WO-A-00/66259
- US-A- 5 759 779
- US-A- 5 763 263
- US-A- 5 798 035

## Beschreibung

Im Gegensatz zu festphasen-immobilisierten miniaturisierten Nukleinsäurebibliotheken, über die eine Vielzahl von Publikationen in Fachzeitschriften und Patenten existieren(S.P.A. Fodor et al. (1991), Light-directed, spatially addressable parallel chemical synthesis. science 2S1.767-773; E.M. Southern et al. (1992), Analyzing and comparing nucleic acid sequences by hybridization to arrays ofoligonucleotides: evaluation using experimental models. Genomics 13:1008-1017; G. McGall. et al. (1996), Light-directed synthesis of high-densily oligonucleotide arrays using semiconductor photoresists.Proc. Natl. Acad. Sci. USA 26:13555-13560, M. Chee et al. (1996), Accessing genetic information with high density DNA arrays. Science 2 74:610-614; 5. Singh-Gasson et al. (1999), Maskless fabrication of light-directed oligonucleotide microarrays using a digital micromirror array. Nat. Biotechnol. 17:974 -978; S.P.A. Fodor et al. (1995), Arrays of materials attached to a substrate. US 5744305; S.P.A. Fodor et al. (1995),Very large scale immobilized polymer synthesis. US 5424186; G.H. MeGall et al. (1995), Spatially-addressable immobilization of oligonucleotides and other biological polymers on surfaces. US 5412087, A.S. Heuermann (1999), Method and device for photolithographic production of DNA, PNA and protein chips. WO 9960156A2, G.H. McGall und N.Q. Nam (2000), Synthesis of oliganucleotide arrays using photocleavableprotecting groups. US 6022963) sind festphasen-immobilisierte stark miniaturisierte Peptid- oder Peptidomimetika-Bibliotheken bislang nur in wenigen Arbeiten beschrieben worden (S.P.A. Fodor et al. (1991), Light-directed, spatially addressable parallel chemical synthesis. Science 25 1:767-773; C.P. Holmes et al. (1995), The use of light-directed combinatorial peptide synthesis in epitope mapping. Biopolymers 3 7:199-211; M.C. Pirrung et al. (1992), Large scale photolithographic solid phase synthesis of polypeptides and receptor binding thereof US 5143854, M.C. Pirrung et al. (1995), Large scale photolithographic solid phase synthesis of an array of polymers. US 5405783).

Einer der Hauptgründe, warum miniaturisierte Peptidbibliotheken, sog. "Peptid-Chips", bislang noch keine weite Verbreitung gefunden haben, ist der relativ große Aufwand bei der Herstellung solcher Anordungen, falls die üblichen, bei "DNA-Chips" verwendeten, photolithographischen Syntheseverfahren auf planaren Trägern zum Einsatz kommen. Diese Verfahren machen sich im Falle der Nukleinsäuren die Tatsache zu Nutze, daß es nur 4 verschiedene natürlich vorkommende Nukleotide (Desoxyadenosin, Desoxycytidin, Desoxyguanosin und Thymidin) für den Aufbau von Desoxyribonukleinsäureoligomeren gibt, daß die Kopplungszeiten beim Aufbau der Oligonukleotide relativ kurz (weniger als 30 min) und die Ausbeuten der einzelnen Kopplungsschritte sehr gut (mehr als 99%) sind. Alle drei Kriterien haben entscheidenden Einfluss auf die Herstellungsdauer für solche Chips und damit die Wirtschaftlichkeit des gesamten Vorgangs.

Bei der photolithographischen Synthese von Oligonukleotiden wird zunächst ein mit einer photolabilen Schutzgruppe vollständig geschützter Träger an den Stellen, an denen z.B. ein Thymidinphosphat aufgebracht werden soll, ortsgerichtet durch Bestrahlung mit Licht entschützt und dann der ganze Träger mit am 5'-OH photolabil geschütztem Thymidinphosphoamidit und geeigneten Kopplungsreagenzien inkubiert. Die Kopplung erfolgt so nur an den gewünschten Stellen und der ganze Prozess muss für die drei anderen Nukleotide wiederholt werden bevor das erste Dinukleotid synthetisiert werden kann. Bei einem Chip, der mit 20-mer Oligonukleotiden belegt wird, sind daher 80 Kopplungs-, Wasch- und Entschützungszyklen erforderlich.

Beim Peptid ist jedoch die Zahl der Komponenten, die zur Kopplung eingesetzt werden können, deutlich höher als im Falle der Nukleinsäuren. Es gibt 20 proteinogene Aminosäuren, einige natürlich vorkommende nicht-proteinogene Aminosäuren (z.B. Ornithin), die gleiche Zahl an entsprechenden D-Aminosäuren sowie eine kontinuierlich steigende Zahl von artifiziellen Aminosäuren, wie z.B. Cyclohexylalanin, Aminoisobuttersäure, Norvalin, etc., ebenfalls jeweils in D- und L-Form. Zusammengenommen kann davon ausgegangen werden, dass zur Zeit etwa 100 verschiedene Aminosäuren zur chemischen Synthese von Peptiden und Peptidomimetika zur Verfügung stehen. Werden - konservativ betrachtet - nur die Hälfte dieser Reagenzien zur Synthese einer Substanzbibliothek eingesetzt, ergeben sich 1.000 Kopplungs- und Entschützungszyklen bei der Synthese einer 20-mer Peptid- bzw. Peptidomimetikabibliothek. Darüberhinaus liefert die Festphasenpeptidsynthese in der Regel Kopplungausbeuten von 85-90% bei Reaktionszeiten von etwa 30 min, so dass üblicherweise mindestens eine Wiederholung des Kopplungsschritts mit frischen Reagenzien notwendig ist, um die erforderlichen Syntheseausbeuten zu erzielen. Dies bedeutet, dass extrem lange Synthesezeiten von Wochen bis hin zu mehreren Monaten für die Herstellung einer auf einem zweidimensionalen Träger iminobilisierten Peptid oder Peptidomimetikabibliothek einkalkuliert werden müssen, wenn nach photolithographischen Verfahren vorgegangen wird. Folglich wurden bislang nur photolitographische Synthesen von kurzen Peptiden geringer Sequenzvariabilität beschrieben (S.P.A. Fodor etal. (1991), Light-directed, spatially addressable parallel chemical synthesis. Science 251:767-773, C.P. Holmes et al. (1995), The use of light directed combinatorial peptide synthesis in epitope mapping. Biopolymers 37:199-211; M.C. Pirrung et al. (1992), Large scale photolithographic solid phase synthesis of polypeptides and receptor binding thereof US 5143854).

Ein alternatives Verfahren, das die Zahl der Arbeitsschritte erheblich reduziert, beruht auf der gleichzeitigen Entschützung aller trägergebundenen Reaktionspartner gefolgt von der parallelen oder sequentiellen ortsgerichteten Applikation der unterschiedlichen Aminosäurereaktionsmischungen in definierte Probenareale. Damit können auch Bibliotheken aus längeren und komplexen Peptiden oder Peptidomimetika in einem akzeptablen Zeitrahmen synthetisiert werden. Hauptproblem hierbei ist allerdings die zu erwartende Kreuzkontamination der Reaktanden, wenn die Probenareale dicht beieinander liegen. Eine dichte Packung der Probenareale wiederum ist wünschenswert, um die Substanzbibliothek ausreichend zu miniaturisieren.

Eine mögliche Lösung des Problems besteht in der Erhöhung der Grenzflächenspannung in den Bereichen zwischen den Probenarealen des planaren Trägers, so dass die Reaktionsmischungen als kleine Tröpfchen im Bereich der Probenareale verharren. Um dieses Ziel zu erreichen, muss die Trägeroberfläche zwischen den Probenarealen fluoralkyliert werden, wie in T.M. Brennan (1995), Method and apparatus for conducting an array of chemical reactions an a support surface, US 5474796; T.M. Brennan (1997), Method and apparatus for conducting an array of chemical reactions an a support surface, EP 703 825B1 und T.M. Brennan (1999), Method and apparatus for conducting an array of chemical reactions an a support surface, US 5985551 beschrieben. Das Verfahren besitzt allerdings den Nachteil, dass die Tropfen ohne schützende Ummantelung stark der Verdunstung ausgesetzt sind, was insbesondere bei sehr kleinen Probenvolumina ein erhebliches Problem darstellen kann.

Eine alternative Lösung des Kreuzkontaminationsproblems besteht in der Verwendung einer porösen Membran, die die Aminosäurereaktionsmischung am Ort der Applikation sofort aufsaugt (R. Frank (1992), Spot synthesis: an easy techniquefor the positionalty addressable, parallel chemical synthesis on a membrane support. Tetra hedron 48:92 17-9232; R. Frank und 5. Güler (1992), Verfahren zur schnellen Synthese von trägergebundenen oder freien Peptiden oder Oligonukleotiden, damit hergestelltes Flachmaterial, Verwendung sowie Vorrichtung zur Durchührung des Verfahrens. WO 92/04366; J. Schneider-Mergener (1994), Verfahren zur Synthese und Selektionierung von Sequenzen aus kovalent verbundenen Bausteinen. WO 94/2052 1). Die ungeordneten Kapillaren in der porösen Membran führen bei zunehmender Miniaturisierung der Probenareale allerdings zu einer Kreuzkontamination und die relativ große Oberfläche der Membran führt auch bei diesem Verfahren zur Verdunstung des Lösungsmittels und damit u.U. auch zu einer Beeinträchtigung der Kopplungsreaktion. Ein weiteres Problem, das beim Einsatz der hier üblicherweise verwendeten Zellulosemembranen auftritt, ist eine zum Teil sehr starke Heterogenität des synthetisierten Produktes. Bei einer massenspektroskopischen Untersuchung des gesamten von einem Probenareal abgelösten Materials findet man sowohl aminoterminal als auch carboxyterminal verkürzte Peptide, die dadurch entstehen, dass aus sterischen Gründen innerhalb der Membran die Synthese zu früh abbricht und dass Kettenneustarts durch Veresterung von Aminosäuren direkt am Zellulosesubstrat auch in späteren Synthesezyklen noch stattfinden können. Es werden insbesondere um 1-4 Aminosäuren carboxyterminal verkürzte Peptide nachgewiesen (D. Goehmann und
A. Frey, unveröffentlichte Resultate). Eine komplette Blockierung der Hydroxylgruppen der Zellulose durch Acetylierung oder ähnliche Maßnahmen zur Verhinderung von Kettenneustarts verbietet sich, da der Träger dadurch eine höhere Löslichkeit in den üblicherweise zur Festphasenpeptidsynthese verwendeten Lösungsmitteln gewinnt und erheblich an mechanischer Stabilität verliert (D. Goehmann und A. Frey, unveröffentlichte Resultate). Ein weiterer Nachteil bei der Verwendung von Membranen besteht in ihren optischen Eigenschaften. Insbesondere Zellulosemembranen weisen eine starke Eigenfluoreszenz im Emissionsbereich vieler handelsüblicher Fluorophore auf, so dass die Empfindlichkeit des Nachweises von Wirkstoffen, die an die Zielmoleküle binden, stark reduziert ist (J. Helfmann, unveröffentlichte Resultate).

Ein weiterer Ansatz zur Lösung des Kreuzkontaminationsproblems bei der Beschickung eines planaren Trägers mit einer Substanzbibliothek besteht in der Verwendung eines Mikroreaktorsystems, bei dem der planare Träger eine Gefäßwand für eine Vielzahl von Reaktionskavitäten und zugleich das Trägermedium für die Zielmoleküle darstellt. Die so allseitig umschlossenen Reaktionskavitäten werden z.B. durch Umpositionieren des Mikroreaktorblocks oder mit Hilfe von Mikrokanälen, elektroosmotischen Pumpen und Mikroventilen im Reaktorblock gezielt mit den gewünschten Reagenzien und Waschlösungen versorgt. Abfallprodukte werden auf analoge Weise entfernt (J.L.Winkler et al. (1995), Very large scale immobilized polymer synthesis using 10 inechanically directed flow paths. US 5384261; P.J. Zanzucchi et al. (1997), Method of synthesis ofplurality of compounds in parallel using a partitioned solid support. US 5643738; P.J. Zanzucchi et al. (1998), Partitioned microelectronic device array. US 5755942; S.C. Cherukuri et al. (1999), Method and system for inhibiting crosscontamination in fluids of combinatorial chemistry device. US 5980704). Ein entscheidender Nachteil solcher Anordnungen ist jedoch ihre Anfälligkeit gegen partikuläre Verunreinigungen in den Reaktionslösungen, insbesondere wenn die Kanäle nur wenige Mikrometer Durchmesser aufweisen und die Flussrichtung der Reagenzien und Abfallprodukte im Mikroreaktorblock mehrfach geändert wird. Die Gefahr einer Verstopfung des Mikroreaktorsystems ist insbesondere bei Kopplungsreaktionen mit Carbodiimiden sehr groß, da die hierbei entstehenden organischen Harnstoffderivate eine starke Tendenz zur Kristallisation aufweisen. Im ungünstigsten Fall muss dann der komplette Mikroreaktorblock ersetzt werden.

Die existierenden bzw. in der Literatur oder Patenten beschrieben Verfahren weisen alle erhebliche Nachteile auf. Es wird entweder keine große Oberfläche und damit keine hohe Nachweisempfindlichkeit bei kleinen äußeren Abmessungen geboten oder die Synthesezeiten sind insgesamt zu lang. Der optische Nachweis wird durch Eigenfluoreszenz stark gestört und Kettenabbrüche führen zu einer inhomogenen Probe. Bei dichter Packung der Substanzbibliothek ist eine Kreuzkontamination zwischen verschiedenen Probenarealen zu beobachten. Bei kleinen Volumina beeinflusst die Verdunstung die Ergebnisse. Die Handhabung ist umständlich oder sehr empfindlich gegen Partikel.

### Erfindungsgemäße Lösung

Die Nachweisempfindlichkeit für die Wechselwirkung (z.B. Bindung) von Wirkstoffen in der Flüssigphase mit festphasen-gebundenen Zielmolekülen wird wesentlich bestimmt durch die Anzahl der festphasen-gebundenen Zielmoleküle. Bei einem ideal planaren Träger ist die zur Bindung zur Verfügung stehende feste Phase auf die äußere Oberfläche des Trägers begrenzt. Ein optisches Nachweissystem (z. B. Fluoreszenzdetektion) ist jedoch immer in der Lage, zusätzlich ein bestimmtes Volumen ober- und unterhalb der äußeren Oberfläche zu erfassen.

Gegenstand der Erfindung ist ein Verfahren und eine Substanzbibliotek,wie in den Ansprüchen 1 und 7 definiert wird. Besondere Ausgestaltungen der Erfindung sind in den Unteransprüchen beansprucht und in der folgenden Beschreibung ausgeführt, ohne dass die Erfindung auf die gezeigten Ausführungsformen beschränkt wäre.

Die Erfindung betrifft eine Substanzbibliothek und ein Verfahren zur Herstellung einer solchen Bibliothek. Unter "Substanzbibliothek" wird dabei eine Bibliothek oder Ansammlung vieler verschiedener, in der Regel aber ähnlicher, Substanzen gleicher Klasse verstanden. Die Bibliothek dient üblicherweise dazu, die dazu gehörenden Substanzen aufgrund irgendeiner Eigenschaft zu durchsuchen und passende Mitglieder oder Zielsubstanzen darin aufzufinden und ggf. zu selektieren. Beispiele für Substanzen, die eine erfindungsgemäße Substanzbibliothek bilden können und die auch als Zielmoleküle bezeichnet werden, sind unter anderem DNA-Sequenzen, Peptide, Polypeptide und peptoide Substanzen.

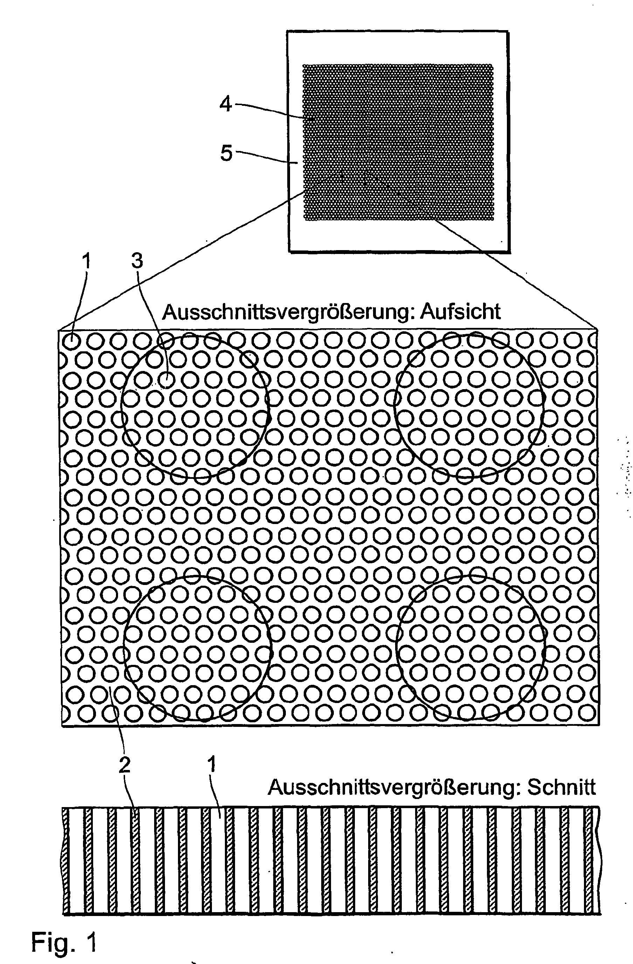

Insbesondere betrifft die Erfindung eine Struktur, die aus Glas, Quarz, Keramik, Kunststoff, Halbmetall oder Metall bestehen kann und durch eine hohe Dichte kleiner Kapillaren [1] (Durchmesser 5-100 *µ*m) eine große innere Oberfläche mit einem Mehrfachen der äußeren Oberfläche besitzt (siehe Figur 1). Die gesamte Dicke dieser Kapillarplatte [4] (Dicke bis zu 10 mm) und damit die gesamte Länge der Kapillaren [1] und deren 15 Oberfläche wird durch eine Nachweisapparatur erfasst. Da die maximale Belegung (Anzahl von Molekülen) der Kapillarplatte [4] durch die Größe der inneren Oberfläche bestimmt ist, und diese im Vergleich zur äußeren Oberfläche um ein Vielfaches (100- bis 1000-fach) größer ist, erhöht sich im gleichen Maße die Nachweisempfindlichkeit bei Nutzung der Kapillarplatte [4] gegenüber einer planaren Probenanordnung.

Überraschenderweise hat sich gezeigt, dass auf einer silanisierten Oberfläche in hoher Qualität peptidische und peptoide Moleküle synthetisiert werden können und die Zahl der Kettenneustarts fast vollständig eliminiert werden kann. Wenn zusätzlich noch ein verlängertes Ankermolekül, das als Abstandshalter dient, zwischen die Oberfläche und das Zielmolekül gekoppelt wird, werden die Kettenabbrüche stark reduziert und die Zugänglichkeit für die Wirkstoffe erheblich verbessert.

Zur Herstellung einer Bibliothek von Zielmoleküllen, von der jede einzelne Spezies oder definierte Mischungen bestimmter Spezies an vorher definierte Bereiche der Kapillarplatte [4] synthetisiert oder gebunden werden, muss zunächst die innere Oberfläche der Kapillarplatte [4] zur kovalenten Bindung der Zielmoleküle befähigt werden. Dies geschieht durch Aufbringen einer Organosilanschicht, die funktionelle Gruppen zur Verankerung von peptidischen oder peptoiden Zielmolekülen aufweist. Vorzugsweise wird dazu ein γ-Aminopropyltrialkoxysilan verwendet, es sind aber auch andere organofunktionelle Silane, wie z.B. γ-Mercaptopropyltrialkoxysilan, für diesen Zweck geeignet. Bei Verwendung von Metall- oder Halbmetallkapillarplatten muss zuvor eine Oxidschicht zur Bindung des Silans mittels Oberflächenoxidation der Kapillarplatte geschaffen werden.

Auf dieser organofunktionalisierten Kapillarplatte [4] wird dann durch ortsabhängiges Aufbringen (z.B. durch Pipettieren) einer Substanz jeweils ein Probenareal [3] angelegt. Bei der Substanz kann es sich im einfachsten Falle um ein Reagenz handeln, das die zur Bindung vorgesehenen funktionellen Gruppen der Organosilanschicht temporär schützt. In einer vorzugsweisen Ausführungsform wird allerdings ein Ankermolekül in den Probenarealen [3] aufgebracht, mit dem die Zielmoleküle zwischen 0,5 bis 20 nm über die innere Oberfläche der Kapillarplatte [4] hinausgehoben werden können, um so eine bessere Wirkstoffbindung zu ermöglichen. Als Ankerreagenz wird z.B. Fluorenylmethoxycarbonyl (FMOC)-geschützter α-Amino-poly(ethylenglycol)-ω-propionsäure-N-hydroxybenzotriazolester verwendet.

Anschließend werden alle nicht mit Schutz- oder Ankerreagentien derivatisierten Bereiche der Kapillarplatte [4] chemisch so abgesättigt, dass dort zu einem späteren Zeitpunkt weder eine Synthese oder Kopplung von Zielmolekülen noch eine unspezifische Bindung von Wirkstoffen erfolgen kann. Die chemische Absättigung der Nicht-Probenareale kann je nach Erfordernis mit hydrophilen, hydrophoben oder oleophoben Gruppen erfolgen. Unter Standardbedingungen erfolgt die Absättigung mit Acetylresten.

In den folgenden Schritten zur erfindungsgemäßen Herstellung einer Substanzbibliothek, die sich auch einer Vielzahl von Probenarealen mit jeweils einer Spezies von Zielmolekülen zusammensetzt, wird die Schutzgruppe auf dem Träger oder den Ankermolekülen in allen Probenarealen [3] synchron entfernt und anschließend die Kopplung oder Synthese der peptidischen und peptoiden Zielmoleküle in den für sie vorgesehenen Probenarealen [3] durchgeführt.

Durch Kenntnis der Substanz, die an jedem Ort auf diese Weise gebunden bzw. synthetisiert wird, ist eine parallele Analyse der Wechselwirkungen von verschiedenen Zielmolekülen mit applizierten Wirkstoffen möglich. Voraussetzung dafür ist, dass keine wechselseitige Verunreinigung beim ortsabhängigen Aufbringen oder Synthetisieren der Zielmoleküle geschieht.

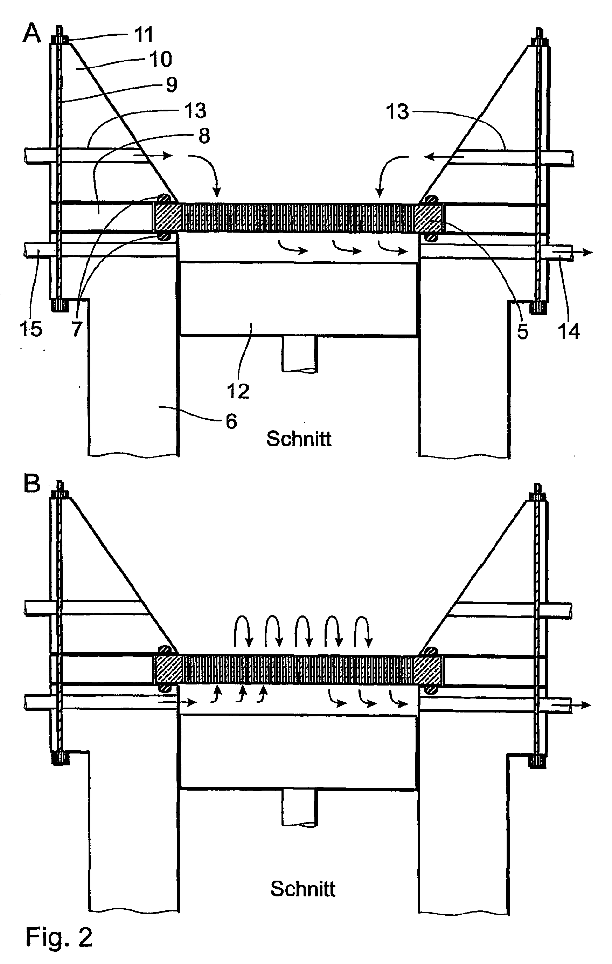

In Weiterführung des Erfindungsgedankens wird dies durch die parallele Anordnung der senkrecht zur Plattenoberfläche verlaufenden Kapillaren [1], die nur zu den äußeren Plattenoberflächen hin offen sind, erreicht. Durch das somit erzielte Fehlen jeglicher Querverbindungen zwischen verschiedenen Probenarealen [3] werden Kreuzkontaminationen auch bei großer Dichte der Probenareale [3] erfolgreich verhindert. Ein Probenareal [3] umfasst mehrere (z.B. bis zu 4.000) Kapillaren [1]. Aufpipettierte Substanzen werden in die Kapillaren [1] gezogen, was je nach Flüssigkeit auch durch Erzeugung eines Unterdrucks auf einer Kapillarplattenseite unterstützt werden kann. Ein Fließen aus den Kapillaren [1] heraus über mehrere Kapillaren [1] hinweg in ein anderes Probenareal [3] ist ausgeschlossen, da die Flüssigkeit durch Kapillarkraft in der Kapillare gehalten wird. Ein Heraustropfen von Lösungen aus den Kapillaren [1] aufgrund sehr geringer Viskosität wird bei diesen Flüssigkeiten durch eine dicht an die Unterseite der Kapillarplatte [4] positionierte Platte [12] (Figur 2) mit hydro- und oleophober Oberfläche wirksam unterdrückt.

In Fortsetzung des Erfindungsgedankens ist aufgrund der nach beiden äußeren. Plattenoberflächen hin offenen Kapillaren [1] auch eine einfache Automatisierung einer in der Kapillarplatte [4] durchgeführten Festphasenpeptidsynthese möglich. Dabei können nach Abschluss eines Kopplungsschrittes die Reagenzien und Abfallprodukte unidirektional durch Anlegen von Vakuum an die Unterseite der Kapillarplatte [4] aus den Kapillaren [1] herausgesaugt und die Kapillaren [1] durch Aufbringen von Waschlösungen auf der Oberseite der Kapillarplatte [4] synchron gespült werden. Für längere Inkubationen und Waschschritte können alle Reagenzien und Waschlösungen, die synchron auf alle Bereiche der Kapillarplatte [4] appliziert werden sollen, auch durch Fluten der gesamten Kapillarplatte [4] von der Unterseite her mit allen Kapillaren [1] in Kontakt gebracht und anschließend durch Anlegen von Unterdruck wieder entfernt werden. Eine Apparatur zur synchronen Applikation von Wasch- und Reagenzlösungen auf alle Bereiche der Kapillarplatte [4] ist in Figur 2 dargestellt.

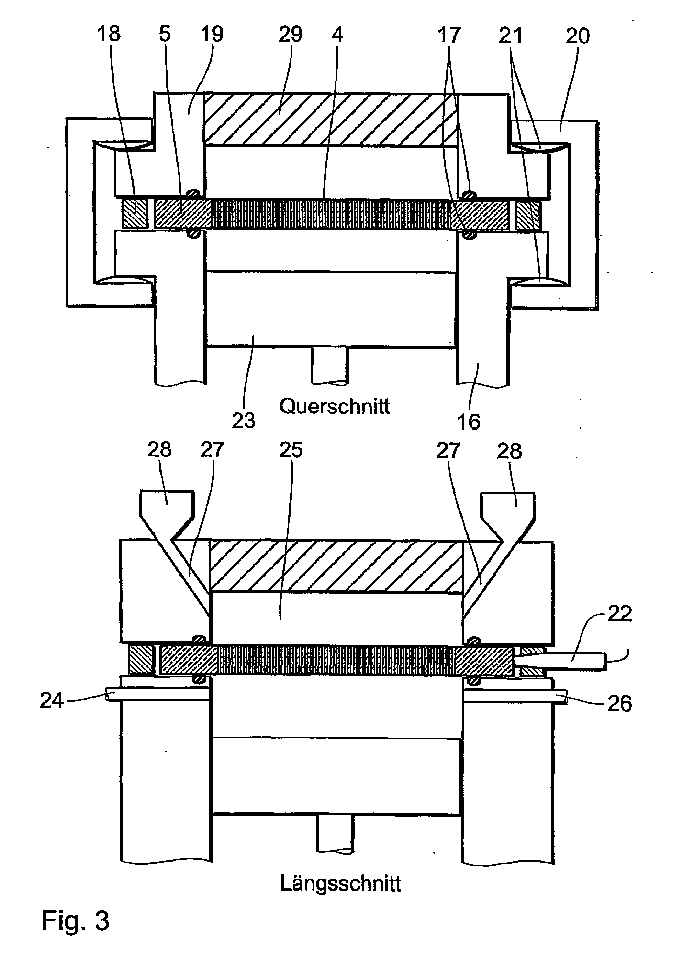

Nachdem auf diese Weise ortsabhängig unterschiedliche Zielmoleküle auf den Probenarealen [3] synthetisiert oder gebunden worden sind, wird der gesamte Träger einem gelösten oder suspendierten partikulären Wirkstoff in der Flüssigphase ausgesetzt, wobei darauf geachtet werden muss, dass beim Einsatz von Suspensionen die Partikelgröße immer deutlich unter dem inneren Durchmesser der Kapillaren [1] liegt. Durch die Redundanz mit vielen Kapillaren je Probenareal stellen einzelne Partikel jedoch kein Verstopfungsrisiko für ein gesamtes Probenareal dar. Die Inkubation derSubstanzbibliothek mit Wirkstofflösungen oder-suspensionen sowie Spülvorgänge davor und danach werden in einer bevorzugten Ausführungsform mit einem einfachen Aufbau durchgeführt, wie er in Figur 3 dargestellt ist.

Die Analyse der Wirkstoffbindung geschieht in einem bevorzugten Ausführungsbeispiel optisch anhand von Fluoreszenzmarkierungen durch Bindung eines Fluorophors an die Wirkstoffe. In einer alternativen Ausführungsform sind die Fluorophore an die Zielmoleküle gebunden. Zum Auslesen wird das einzelne Probenvolumen, das sich aus der Dicke der Kapillarplatte und dem Probenbereich [3] zusammensetzt, sowohl von Anregungslicht vollständig durchsetzt als auch von der Detektionsoptik vollständig erfasst. Die Vielzahl der Probenorte wird durch zeitlich sequentielles Abrastern erfasst, wobei entweder die Kapillarplatte zweidimensional unter der ortsfesten optischen Anordnung bewegt wird oder der Strahlengang über die Kapillarplatte bewegt wird. Die Verwendung von Quarz oder Glas als Material für die Kapillarplatte minimiert hier erfindungsgemäß den Einfluss der

**Detaillierte Beschreibung der erfindungsgemäßen Lösung in einer bevorzugten Ausführungsform**

Es wird beschrieben wie die gesamte Kapillarplatte vorbehandelt wird. Anschließend werden die Probenareale erzeugt und darauf die Substanzen synthetisiert. Für alle Vorgänge, der die Kapillarplatte vollständig ausgesetzt wird, wie z.B. Waschen, ist eine automatisierte Vorrichtung beschrieben. Diese Vorrichtung ist mit einem Pipettierroboter kombiniert, so dass die Kapillarplatte in der gleichen Vorrichtung gewaschen und entschützt werden kann sowie die einzelnen Syntheseschritte durchgeführt werden können. Zur Untersuchung der Bindung eines Wirkstoffs an die Substanzbibliothek wird eine weitere Vorrichtung beschrieben, in die die Kapillarplatte eingelegt wird und mit dem Wirkstoff in Kontakt gebracht wird. Hierin wird gleichzeitig der optische Nachweis der Bindung durchgeführt.

### Derivatisierung der Kapillarplatte zur Aufnahme der Substanzbibliothek

Die Kapillarplatte wird durch Ätzen aus homogenem Glas, in einem alternativen Verfahren aus heterogenem Glas, hergestellt. Das Oxidieren von Silizium und Metalloberflächen und Silanisieren von Glas-, Quarz-, Silizium-, Keramik- sowie oxidierten Silizium- oder Metalloberflächen ist Stand der Technik und wurde in der Fach- und Patentliteratur bereits vielfach beschrieben (z.B. zur Oberflächenoxidation eines Siliziumträgers: A.W. Flounders et al. (1997), Patterning of immobilized antibody layers via photolithography and oxygen plasma exposure. Biosensors Bioelectronics 12:447-456; z.B. zur Silanisierung: M. Lynn (1975), Inorganic support intermediates:covalent coupling of enzymes on inorganic supports. In: "Immobilized Enzymes, Antigens, Antibodies and Peptides", H.H. Weetall, Hrsg., Marcel Dekker, New York, NY, USA, pp. 1-48; H.M. Weetall (1976), Covalent coupling methods for inorganic support materials. Methods EnzymoL 44:134-148).

In dem bevorzugten Ausführungsbeispiel wird dazu eine unbehandelte Kapillarplatte [4] mit Oberflächenoxid-, Keramik- oder Glasbeschichtung entweder in einer oxidierenden Säure, wie z.B. Chromschwefelsäure oder heißer konzentrierter Salpetersäure, für eine Stunde von adsorbierten organischen Verbindungen befreit, fünf- bis zehnmal durch Durchsaugen von hochreinem Wasser (auf einer Nutsche, Glasfritte oder einer speziell für die Kapillarplatte [4] entwickelten Spülvorrichtung (Figur 2) gewaschen und bei 250 °C getrocknet.

Alternativ können anheftende organische Verbindungen durch Pyrolyse bei 500°C in einer Sauerstoffatmosphäre entfernt werden. Nach dem Abkühlen wird die Kapillarplatte [4] für 18 Stunden bei Raumtemperatur in einer Lösung von 2% (v/v) γ-Aminopropyltriethoxysilan in Wasser/Aceton (1:1) beschichtet, zehnmal durch Durchsaugen von Aceton gewaschen, trocknen lassen und über Nacht bei 120 °C gesintert.

### Definition der Probenareale

Die Definition der Probenareale [3] ist im bevorzugten Ausführungsbeispiel ein zweistufiges Verfahren, wobei zuerst mit Hilfe eines programmierbaren Pipettierroboters Portionen einer Reaktionslösung mit Ankermolekülen in bestimmte Regionen der Kapillarplatte [4] pipettiert werden, die damit zu Probenarealen [3] werden. Im zweiten Schritt werden alle reaktiven Gruppen in den Nicht-Probenarealen mit Acetylresten abgesättigt.

Dazu werden jeweils ca. 0,4 bis 4000 nl einer Lösung, die einen zweifachen molaren Überschuß - bezogen auf die Anzahl der Aminofunktionen im entsprechenden Bereich der derivatisierten Kapillarplatte [4] - an α-FMOC-Amino-poly(ethylenglycol)propionsäure-ω-N-hydroxybenzotriazolester in N-Methylpyrrolidon enthält, in die künftigen Probenareale [3] der Kapillarplatte [4] pipettiert, für beispielsweise vier Stunden bei Raumtemperatur inkubiert, jeweils dreimal durch Durchsaugen von Dimethylformamid und absolutem Ethanol gewaschen und unter Reinraumbedingungen im Luftstrom getrocknet. Zur Erhöhung der Kopplungsausbeute kann der gesamte Kopplungs- und Waschzyklus ein bis dreimal wiederholt werden. Der α-FMOC-Amino-poly(ethylenglycol)propionsäure-ω-N-hydroxybenzotriazolester wird unmittelbar zuvor z.B. durch Umsetzung von α-FMOC-Aminopoly(ethylenglycol)propionsäure mit 2-(1H-Benzotriazol-1-yl)-1,1,3,3-tetramethyluroniumhexafluorophosphat (HBTU) (z.B. im Verhältnis 1:1,2) oder in situ durch Umesterung von z.B. α-FMOC-Amino-poly(ethylenglycol)propionsäure-ω-N-hydroxysuccinimidester mit wasserfreiem N-Hydroxybenzotriazol und Diisopropylcarbodiimid (im Verhältnis 1:0,5:0,5) in N-Methylpyrrolidon hergestellt.

Im anschließend Absättigungsverfahren wird die gesamte Kapillarplatte [4] z.B. jeweils dreimal für je eine Stunde bei Raumtemperatur in fünf- bis zehnfachen inneren Plattenvolumen einer Lösung von 2% (v/v) Acetanhydrid, 1 % (v/v) Diisopropylethylamin in Dimethylformamid inkubiert, wobei die Platte alle 15 min so in der Lösung bewegt wird, daß sich die Reaktionslösung in den Kapillaren [1] austauscht. Danach wird die Kapillarplatte [4] auf einer Fritte oder Nutsche je dreimal mit Dimethylformamid und Ethanol gewaschen und im Luftstrom getrocknet.

Im folgenden Entschützungsverfahren wird die abgesättigte Kapillarplatte [4] dreimal für je fünf Minuten bei Raumtemperatur in fünf bis zehn inneren Plattenvolumen einer Lösung von 20% (v/v) Piperidin in Dimethylformamid inkubiert. Danach wird die Kapillarplatte [4] wie oben beschrieben gewaschen und abschließend getrocknet.

### Herstellung der Substanzbibliothek in den Probenarealen

Die Synthese der Substanzbibliothek von peptidischen oder peptoiden Zielmolekülen verläuft semi- oder vollautomatisch nach dem Standard-FMOC-Syntheseverfahren für Zellulosefilter-immobilisierte Peptidbibliotheken (R. Frank (1992), Spot synthesis: an easy technique for the positionally addressable, parallel chemical synthesis on a membrane support. Tetra hedron 48:9217-9232), wobei die Lösungen, welche die aktivierten, aminoterminal und Seitenketten-geschützten Aminosäuren enthalten, mit Hilfe eines Pipettierroboters in die entsprechenden Probenareale [3] der Kapillarplatte [4] pipettiert und dort durch Kapillarkräfte in die Kapillaren [1] gezogen und für den Verlauf der Kopplungsreaktion inkubiert werden. Beim vollautomatischen Verfahren (siehe Figur 2) wird die Kapillarplatte [4] mit dem 5-fachen inneren Plattenvolumen an Dimethylformamid innerhalb von 30 sec überschichtet und die Reaktionsgemische gefolgt von der darübergeschichteten Waschlösung mit Unterdruck abgesaugt. Dann werden entweder die nicht reagierten Aminofunktionen mit Hilfe von Acetanhydrid blockiert oder nach weiterem Waschen der Kapillarplatte [4] in Dimethylformamid und Ethanol der gesamte Kopplungszyklus noch ein- bis dreimal wiederholt, bevor die nicht reagierten Aminofunktionen blockiert und anschließend die FMOC-Schutzgruppen in den Probenarealen [3] der gesamten Kapillarplatte [4] entfernt werden. Dieser Synthesezyklus wird so lange wiederholt, bis die Peptide in der gewünschten Länge erhalten werden. Dann werden die Seitenketten der Aminosäuren entschützt, die gesamte Kapillarplatte [4] mit Dimethylformamid und Ethanol gewaschen, unter Reinraumbedingungen im Luftstrom getrocknet, unter Schutzgas (z.B. Argon oder Stickstoff) in eine Kunststofffolie eingeschweißt und bei -70 °C in Gegenwart von Trockenmittel gelagert bis sie für die weitere Untersuchung der Bindung von Wirkstoffen an die Substanzen auf der Kapillarplatte benötigt werden.

Alternativ dazu können auf die freien Aminofunktionen in den Probenarealen [3] der Kapillarplatte [4] auch z.B. Bromacetylreste zur Kopplung von thiol-haltigen Peptiden oder Aldehydreste zur Bindung von Proteinen aufgebracht werden. Welche Biokonjugatchemie hier optimal geeignet ist, hängt von den individuellen Erfordernissen der zu koppelnden peptidischen oder peptoidischen Zielmoleküle ab und kann an dieser Stelle nicht allgemeingültig beschrieben werden.

### Vorrichtung zum vollautomatischen Waschen, Absättigen und Entschützen

Das vollautomatische Waschen, Absättigen und Entschützen ist die bevorzugte Ausführungsform bei der Herstellung der Substanzbibliothek. Es ist gegenüber einem manuellen Verfahren bevorzugt, da es Arbeitszeit und Reagenzien spart und die gesamte Synthese verkürzt und kosteneffizienter gestaltet (Figur 2).

Zur Montage wird die Kapillarplatte [4] auf das Unterteil eines Synthesetisches [6] gelegt, der fest mit einem Pipettierroboter verbunden ist. Dabei kommen die Randbereiche [5] der Kapillarplatte auf einer weichen, lösungsmittelresistenten Dichtung [7] zu liegen. Anschließend werden Abstandhalter [8] rings um die Kapillarplatte [4] dicht an die Randbereiche [5] herangeschoben und durch Gewindebolzen [9] gegen Verschieben gesichert, so daß die Kapillarplatte [4] auf dem Probentisch ortsfest positioniert ist. Danach wird das Oberteil des Synthesetisches [10] auf die Gewindebolzen [9] gesteckt und mit Hilfe einer Mutter [11] gegen die Kapillarplatte [4] und den Abstandhalter [8] bzw. das Unterteil des Synthesetisches [6] gezogen, so daß die im Ober- und Unterteil des Synthesetisches [10 und 6] befindlichen Dichtungen [7] zusammengepreßt werden und die Kapillarplatte [4] zur Seite hin abdichten.

Für das Beschicken der Probenareale [3] wird der hydrophob und oleophob beschichtete Kolben [12] an die Unterseite der Kapillarplatte [4] herangeschoben, so daß keine Reaktionslösung während der Kopplungsreaktion nach unten aus der Kapillarpiatte [4] austreten kann.

Zum Waschen nach Beendigung der Kopplungsreaktion wird die gesamte Kapillarplatte [4] aus den Waschlösungszuflüssen [13] mit Waschlösung überschichtet, der Kolben [12] nach unten bewegt und zur Entfernung der durch die Kapillarplatte [4] gesaugten Lösungen am Ablauf [14] Vakuum angelegt. Dann wird der Kolben [12] wieder an die Unterseite der Kapillarplatte [4] herangeschoben, die Kapillarplatte [4] wieder mit Waschlösung überschichtet und der Waschvorgang beliebig wiederholt (Figur 2A). Nach Abschluß des Waschvorgangs wird so lange Luft durch die Kapillarplatte [4.] gesaugt, bis alle Lösungsmittelreste verdunstet sind. Dann wird der Kolben wieder an die Kapillarplatte [4] herangeschoben und ein erneuter Pipettierzyklus durchgeführt, die Platte abgesättigt oder entschützt. Zum Absättigen nicht reagierter Aminofunktionen und zum Entschützen von Aminofunktionen wird der Kolben [12] nach unten geschoben und die Kavität, die durch Zurückziehen des Kolbens [12] unterhalb der Kapillarplatte [4] entsteht, mit der gewünschten Reaktionslösung durch den Reagenzienzufluß [15] befüllt. Bei verschlossenem Ablauf [14] und Reagenzienzufluß [15] wird der Kolben für die Dauer der Reaktion langsam auf- und abbewegt, so daß die Reaktionslösung in gleichmäßigem Fluß durch die Kapillaren [1] strömt und die Reaktion stattfinden kann (Figur 2B). Anschließend wird die Kapillarplatte [4] wie oben beschrieben gewaschen.

### Vorrichtung zur Bindung von Wirkstoffen an die auf der Kapillarplatte immobilisierte Substanzbibliothek

Zur Untersuchung der Bindung von Wirkstoffen an die auf der inneren Oberfläche der Kapillarplatte [4] befindliche Substanzbibliothek ist eine Vorrichtung notwendig, mit der eine Wirkstofflösung oder -suspension gleichmäßig durch alle Kapillaren [1] gespült werden kann, so daß jede Wirkstoffkomponente mit jeder Zielmolekülspezies in Kontakt kommen kann (Figur 3).

Im bevorzugten Ausführungsbeispiel wird zur Montage die Kapillarplatte [4] auf das Unterteil des Inkubationstisches [16] gelegt. Dabei kommen die Randbereiche [5] der Kapillarplatte auf einer weichen Dichtung [17] zu liegen. Anschließend werden Abstandhalter [18] rings um die Kapillarplatte [4] gelegt, das Oberteil des Inkubationstisches [19] aufgesetzt und durch eine Klammer [20] mittels Andruckfedern [21] auf die Kapillarplatte [4] und den Abstandhalter [18] bzw. das Unterteil des Inkubationstisches [16] gedrückt, so daß die im Ober- und Unterteil des Inkubationstisches [19 und 16] befindlichen Dichtungen [17] zusammengepreßt werden 5 und die Kapillarplatte [4] zur Seite hin abdichten. Dann wird ein Ultraschallgeber [22] in eine Ausparung des Abstandhalters [18] geschoben, bis er Kontakt mit dem Randbereich [5] der Kapillarplatte [4] hat.

Zur Beschickung der Vorrichtung wird der Kolben [23] nach unten geschoben und die Kavität, die durch Zurückziehen des Kolbens [23] unterhalb der Kapillarplatte [4] entsteht, mit der gewünschten Wirkstofflösung oder -suspension durch den Einfüllstutzen [24] befüllt. Das Volumen der Wirkstofflösung oder-suspension sollte mindestens das 1,5-fache des inneren Volumens der Kapillarplatte [4] plus das Volumen der über der Kapillarplatte [4] befindlichen Kavität [25] betragen.

Zur Inkubation wird bei verschlossenem Ablauf [26] und Einfüllstutzen [24] der Kolben [23] langsam nach oben bewegt, so daß die Luft aus den Kapillaren [1] in die Kavität [25] entweichen kann. Noch in Kapillaren [1] eingeschlossene Luft kann durch Beschallung mit dem Ultraschallgeber [22] entfernt werden. Wenn der Kolben [23] bis an die Kapillarplatte [4] herangeschoben wird, füllt sich die Kavität [25] vollständig mit Wirkstofflösung oder -suspension und sowohl die Luft als auch überzählige Wirkstofflösung bzw. -suspension weichen durch seitliche Kanäle [27] in die Ausgleichsgefäße [28] aus. Durch wiederholtes Heben und Senken des Kolbens [23] mit kurzem Hub wird dann die Lösung oder Suspension in den Kapillaren [1] mit der in der Kavität [25] befindlichen Lösung oder Suspension gemischt und noch in der Kavität [25] befindliche Luftblasen schrittweise in die Ausgleichsgefäße [28] vertrieben.

Zum Entleeren der Vorrichtung wird der Kolben [23] so weit zurückgeschoben, daß der Ablauf [26] freikommt, durch den dann die Wirkstofflösung oder -suspension durch Anlegen von Unterdruck entfernt wird.

Das Waschen der Vorrichtung erfolgt in analoger Weise unter Verwendung geeigneter Waschlösungen.

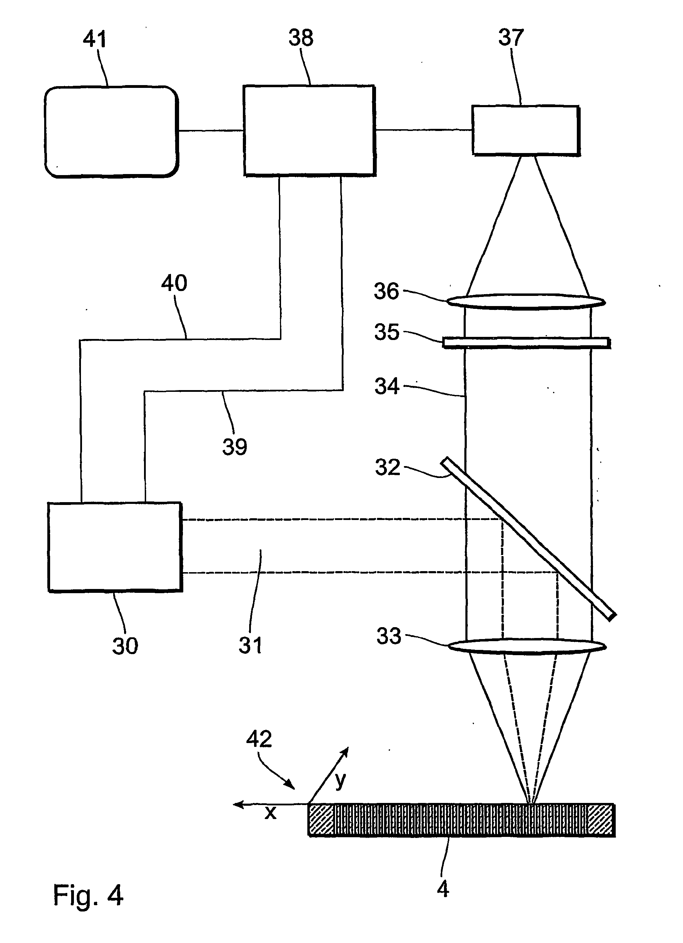

Die ortsaufgelöste Detektion der Wirkstoffreaktion mit den Zielmolekülen kann ebenfalls in der Vorrichtung erfolgen, da die über der Kapillarplatte [4] befindliche Kavität [25] nach oben durch ein transparentes Fenster [29] begrenzt wird und damit beispielsweise die Bindung oder die Abspaltung von Fluorophoren in der Vorrichtung mit Hilfe der in Figur 4 beschriebenen Meßeinrichtung detektiert bzw. verfolgt werden kann.

### Vorrichtung zum Nachweis der Bindung von Wirkstoffen an Zielmoleküle

Nachweisverfahren für die Bindung von Molekülen sind in der Literatur und in Patenten vielfältige beschrieben, sie basieren meist auf der Verwendung von radioaktiven oder optischen Markern. Für den Nachweis von Bindungen in der Kapillarplatte werden optische Methoden die eine Durchdringung der Stege zwischen den Kapillaren erlauben, verwendet. Die vielfältigsten optischen Methoden basieren auf Fluoreszenzmarkierungen (z.B. M.V. Rogers (1997), Light on highthroughput screening: fluorescence-based assay technologies, Drug discovery Today 2:156-1 60). Die Fluoreszenzmarkierung wird durch Kopplung eines Fluorophors entweder an die Zielmoleküle oder an die Wirkstoffe hergestellt. Zum

Auslesen wird im bevorzugten Ausführungsbeispiel das einzelne Probenvolumen, das dem Probenareal [3] über die gesamte Dicke der Kapillarplatte entspricht, sowohl von Anregungslicht vollständig durchsetzt, als auch von der Detektionsoptik vollständig erfaßt. Um ein Übersprechen von benachbarten Probenarealen zu verhindern, wird zu einem Zeitpunkt nur ein Probenort beleuchtet und die Detektion nur auf einen Probenort begrenzt. Die Vielzahl der Probenorte wird durch zeitlich sequentielles Abrastern erfasst. Die Messung an einem Probenort kann sowohl kontinuierlich als auch mit repetierenden Pulsen geschehen. Im ersten Fall wird mit einer kontinuierlich abstrahlenden bandpaßgefilterten Lichtquelle oder einem Laser bestrahlt, wobei das schmalbandige Anregungslicht an die Absorption des Fluoreszenzmarkers angepaßt ist. Die Detektion geschieht schmalbandig im Bereich der Fluoreszenzemission des Fluoreszenzmarkers miteinem geeigneten Photodetektor. Bei der in Figur 4 dargestellten gepulsten Fluoreszenzanregung wird ein Laser [30] zur Beleuchtung eingesetzt, dessen Pulsdauer mindestens ein Drittel der

Lebensdauer des Markierungs-Fluorophors beträgt. Der kollimierte Strahl des Lasers [31] wird durch einen wellenlängenselektiven Strahlteiler [32] in Richtung auf die Kapillarplatte [4] reflektiert. Durch ein Objektiv [33] wird der Strahl so auf ein Probenareal [3] der Kapillarplatte [4] fokussiert, daß das gesamte Probenvolumen eines Probenareals [3] durchstrahlt wird. Das isotrop abgestrahlte Fluoreszenzlicht [34] der Fluroreszenzmarkierung wird durch das gleiche Objektiv [33] kollimiert und passiert den selektiven Strahlteiler [32] aufgrund der größeren Wellenlänge der Fluoreszenz. Durch ein schmalbandiges Filter [35] wird Streulicht des Anregungslasersstarkunterdrückt, das Fluoreszenzlicht bei möglichst hoher Transmission aber durchgelassen. Mit einem weiteren Objektiv [36] wird das Fluoreszenzlicht auf den Photodetektor [37] fokussiert. Durch eine zeitaufgelöste Detektion mit einer Anstiegszeit von kleiner als einem Drittel der Fluoreszenzlebensdauer des Fluorophors kann das Fluoreszenzsignal besonders vorteilhaft in einer Zeittor-Integrationstechnik mit einem Boxcar-Integrator [38] nach Abklingen sowohl des elastischen Streulichts, als auch von Raman-gestreutem Licht erfaßt werden. Das Zeittor wird sinngemäß ca. nach der zweifachen Laserpulsdauer geöffnet und bleibt für ca. die zweifache Fluorophorlebensdauer offen.

Ein Triggersignal vom Laser [39] liefert die Zeitbasis für das Zeittor. Mit Hilfe eines der Laserpulsenergie proportionalen Signals vom Laser [40] kann über einen zweiten Kanal des Boxcar-Integrators [38] eine Normierung des Fluoreszenzlichts und damit eine Korrektur für Schwankungen der Laserpulsenergie durchgeführt werden. In einem Rechner [41] werden alle Daten gesammelt und es kann abschließend eine Auftragung der Fluoreszenzintensität über dem jeweiligen Probenort dargestellt werden und eine Zuordnung der detektierten Signale zu den Zielmolekülen erfolgen.

### Beschreibung der Figuren

Figur 1 enthält eine schematische Darstellung einer Kapillarplatte [4] in Aufsicht und als Schnitt. Die einzelnen Kapillaren [1] sind durch Stegbereiche [2] voneinander getrennt. Die Probenareale [3] umfassen eine Vielzahl von Kapillaröffnungen. Der Randbereich der Platte [5] enthält keine Kapillaren.

Figur 2 zeigt eine Vorrichtung zum vollautomatischen Waschen, Absättigen und Entschützen der Kapillarplatte [4] während der Synthese der Substanzbibliothek. Dabei ist die Kapillarplatte [4] zwischen Oberteil [10] und Unterteil [6] des Synthesetisches eingesetzt und mit Hilfe entsprechender Dichtungen [7] und Abstandhalter [8] über einen Gewindebolzen [9] mit Mutter [11] in ihrer Position fixiert. Im Oberteil des Synthesetisches [10] befinden sich Waschlösungszuflüsse [13], im Unterteil [6] Reagenzienzufluß [15] und Ablauf [14]. Ein Kolben [12] kann im Unterteil des Synthesetisches [6] bewegt und bis an die Unterseite der Kapillarplatte [4] geschoben werden.

Figur 3 zeigt eine Vorrichtung zur Bindung von Wirkstoffen an die auf der Kapillarplatte immobilisierte Substanzbibliothek. Die Kapillarplatte [4] ist zwischen Oberteil [19] und Unterteil [16] des Inkubationstisches eingesetzt und mit Hilfe entsprechender Dichtungen [17] und Abstandhalter [18] durch eine Klammer [20] mit Andruckfeder [21] in ihrer Position fixiert. Ein Ultraschallgeber [22] ist seitlich an der Kapillarplatte [4] angebracht. Im Unterteil des Inkubationstisches befinden sich Einfüllstutzen [24] und Ablauf [26] für die Wirkstofflösungen, die mit einem Kolben [23] durch die Kapillarplatte [4] in die obere Kavität [25] gedrückt werden können. Seitliche Kanäle [27] führen aus der Kavität [25] in ein Ausgleichsgefäß, das zum Auffangen verdrängter Luft und überschüssiger Wirkstofflösung dient. Die Kavität [25] ist oben mit einem transparenten Fenster [29] abgeschlossen, so daß eine direkte optische Detektion von Reaktionen in der Vorrichtung möglich ist.

Figur 4 enthält eine schematische Darstellung einer Vorrichtung zum Nachweis der Bindung von Wirkstoffen an die Substanzbibliothek mittels optischer Verfahren. Fluorophormarkierte Zielmoleküle auf der Kapillarplatte [4] werden durch einen von einem gepulsten Diodenlaser [30] erzeugten Laserstrahl [31], der durch einen wellenlängenselektiven Strahlteiler [32] und ein Objektiv [33] auf das Probenareal gelenkt wird, zur Fluoreszenz angeregt. Das abgestrahlte Fluoreszenzlicht [34] durchdringt den Strahlteiler [32], einen Bandpaßfilter [35] und ein weiteres Objektiv [36], bevor es auf den Photodetektor [37] auftrifft. Für die Integration des Fluoreszenzsignals innerhalb eines Zeittores erhält ein Boxcar-Integrator [38] vom Laser ein zeitliches Triggersignal [39] und zur Normierung des Fluoreszenzsignals ein der Laserenergie proportionales Signal [40]. Von einem Rechner [41] wird ein beweglicher Probentisch [42] so angesteuert, daß nacheinander die Probenareale gemessen werden können. Das normierte Fluoreszenzsignal wird über dem Probenort dargestellt.

### Legende

- 1: Kapillare
- 2: Stegbereich zwischen Kapillaren
- 3: Probenareal
- 4: Kapillarplatte
- 5: Randbereich der Kapillarplatte
- 6: Unterteil des Synthesetisches
- 7: Lösungsmittelresistente Dichtung
- 8: Abstandhalter
- 9: Gewindebolzen
- 10: Oberteil des Synthesetisches
- 11: Mutter
- 12: Kolben
- 13: Waschlösungszufluß
- 14: Ablauf
- 15: Reagenzienzufluß
- 16: Unterteil des Inkubationstisches
- 17: Dichtung
- 18: Abstandhalter
- 19: Oberteil des Inkubationstisches
- 20: Klammer
- 21: Andruckfeder
- 22: Ultraschallgeber
- 23: Kolben
- 24: Einfullstutzen
- 25: Kavität
- 26: Ablauf
- 27: Kanal
- 28: Ausgleichsgefäß
- 29: Transparentes Fenster
- 30: Gepulster Diodenlaser
- 31: Laserstrahl
- 32: Strahlteiler
- 33: Objektiv
- 34: Abgestrahltes Fluoreszenzlicht
- 35: Bandpaßfilter
- 36: Objektiv
- 37: Photodetektor
- 38: Boxcar-Integrator
- 39: Triggersignal vom Laser
- 40: Der Laserpulsenergie proportionales Signal vom Laser
- 41: Rechner
- 42: Beweglicher Probentisch

Im folgenden werden noch einige bevorzugte Ausführungsformen der erfindungsgemäßen Anordnung bzw. des erfindungsgemäßen Verfahrens angegeben.
- A.: Verfahren und Vorrichtung zur Untersuchung der molekularen Wechselwirkung von löslichen oder suspendierbaren Wirkstoffen mit festphasengebundenen peptidischen oder peptoiden Zielmolekülen dadurch gekennzeichnet, daß die dafür notwendige Oberfläche innerhalb einer Platte in Form von Kapillaren geschaffen wird. Die Kapillaren von einer Plattenseite zur gegenüberliegenden Seite durchgehen, dabei aber untereinander nicht verbunden sind.
- B.: Verfahren und Vorrichtung, wie gemäß A definiert, die dadurch gekennzeichnet sind, daß eine Vielzahl von parallelen Kapillaren in der Platte angeordnet sind, die Flüssigkeiten durch Kapillarkraft in die Kapillaren ziehen und darin halten.
- C.: Verfahren und Vorrichtung wie in den vorherigen Abschnitten definiert, die dadurch gekennzeichnet sind, dass die Kapillarplatte aus einem der Materialien Glas, Quarz, Silizium, Kunststoff, Halbmetall, Keramik, Metall oder einer Kombination dieser Materialien hergestellt ist.
- D.: Verfahren und Vorrichtung wie in den vorherigen Abschnitten definiert, die dadurch gekennzeichnet sind, dass die innere Oberfläche zur kovalenten Bindung von Zielmolekülen befähigt wird oder eine Synthese der Zielmolekülen erfolgt.
- E.: Verfahren und Vorrichtung wie in den vorherigen Abschnitten definiert, die dadurch gekennzeichnet sind, dass die innere Oberfläche durch eine Organosilanschicht, die funktionelle Gruppen zur Verankerung von peptidischen oder peptoiden Zielmolekülen aufweist, bedeckt ist.
- F.: Verfahren und Vorrichtung wie in den vorherigen Abschnitten definiert, die dadurch gekennzeichnet sind, dass zur Silanisierung γ-Aminopropyltrialkoxysilan oder andere organofunktionelle Silane verwendet werden.
- G.: Verfahren und Vorrichtung wie in den vorherigen Abschnitten definiert, die dadurch gekennzeichnet sind, dass bei Verwendung von Metall- oder Halbmetallkapillarplatten vor der Silanisierung eine Oxidschicht zur Bindung des Silans mittels Oberflächenoxidation der Kapillarplatte geschaffen wird.
- H.: Verfahren und Vorrichtung wie in den vorherigen Abschnitten definiert, die dadurch gekennzeichnet sind, dass in der Kapillarplatte örtlich begrenzte Probenareale angelegt werden.
- I.: Verfahren und Vorrichtung wie in den vorherigen Abschnitten definiert, die dadurch gekennzeichnet sind, dass eine Vielzahl von Probenarealen jeweils mit einem Durchmesser von bis zu 2.000 um oder einer Anzahl von bis zu 4.000 benachbarten. Kapillaren auf einer Kapillarplatte angelegt werden.
- J.: Verfahren und Vorrichtung wie in den vorherigen Abschnitten definiert, die dadurch gekennzeichnet sind, dass die Kapillaren eine Länge sowie die Kapillarplatte eine Dicke von 100 bis 2.000 µm besitzen.
- K.: Verfahren und Vorrichtung wie in den vorherigen Abschnitten definiert, die dadurch gekennzeichnet sind, dass durch Aufpipettieren einer Substanz auf die Kapillarplatte die funktionelle Gruppe der Organosilanschicht temporär geschützt wird. Der so geschützte Bereich stellt ein Probenareal dar.
- L.: Verfahren und Vorrichtung wie in den vorherigen Abschnitten definiert, die dadurch gekennzeichnet sind, dass durch Aufpipettieren eines Reagenz auf die Kapillarpiatte ein geschütztes Ankermolekül an jeder Bindungsstelle des Probenareals entsteht, wobei das Ankermolekül gleichzeitig als Abstandshalter zur inneren Oberfläche dient.
- M.: Verfahren und Vorrichtung wie in den vorherigen Abschnitten definiert, die dadurch gekennzeichnet sind, dass durch Aufpipettieren von Fluorenylmethoxycarbonyl (FMOC)-geschützter α-Amino-poly(ethylenglycol)-ω-propionsäure-N-hydro-xybenzotriazolester ein geschütztes Probenareal angelegt wird.
- N.: Verfahren und Vorrichtung wie in den vorherigen Abschnitten definiert, die dadurch gekennzeichnet sind, dass alle nicht geschützten Bereiche der Kapillarplatte chemisch abgesättigt werden.
- O.: Verfahren und Vorrichtung wie in den vorherigen Abschnitten definiert, die dadurch gekennzeichnet sind, dass die Schutzgruppen in den Probenarealen der gesamten Kapillarplatte entfernt werden.
- P.: Verfahren und Vorrichtung wie in den vorherigen Abschnitten definiert, die dadurch gekennzeichnet sind, dass Peptide oder peptoide Zielmoleküle ortsabhängig sequentiell aus Aminosäuren in den Probenarealen der Kapillarplatte synthetisiert werden.
- Q.: Verfahren und Vorrichtung wie in den vorherigen Abschnitten definiert, die dadurch gekennzeichnet sind, dass die vollständigen peptiden oder peptoiden Zielmoleküle ortsabhängig in den Probenarealen der Kapillarplatte gebunden werden.
- R.: Verfahren und Vorrichtung wie in den vorherigen Abschnitten definiert, die dadurch gekennzeichnet sind, dass durch die ortsabhängig unterschiedlichen Sequenzen von peptiden oder peptoiden Zielmolekülen eine Substanzbibliothek aufgebaut wird.
- S.: Verfahren und Vorrichtung wie in den vorherigen Abschnitten definiert, die dadurch gekennzeichnet sind, dass auf mindestens einer Seite der Kapillarplatte ein mit einem Ventil versehenes geschlossenes und variables Volumen existiert, in das Flüssigkeiten mit löslichen oder suspendierbaren Wirkstoffen sowie andere Flüssigkeiten und Gase eingelassen werden können und abgesaugt werden können.
- T.: Verfahren und Vorrichtung wie in den vorherigen Abschnitten definiert, die dadurch gekennzeichnet sind, dass durch Ankopplung von Ultraschall an die Kapillarplatte Gasblasen gelöst werden können.
- U.: Verfahren und Vorrichtung wie in den vorherigen Abschnitten definiert, die dadurch gekennzeichnet sind, dass durch Ankopplung von Ultraschall die Reaktionsgeschwindigkeiten in den Kapillaren erhöht werden.
- V.: Verfahren und Vorrichtung wie in den vorherigen Abschnitten definiert, die dadurch gekennzeichnet sind, dass die Bindung der Wirkstoffe an die Zielmoleküle nachgewiesen wird.
- W.: Verfahren und Vorrichtung wie in den vorherigen Abschnitten definiert, die dadurch gekennzeichnet sind, dass eine Veränderung der Zielmoleküle durch die Wirkstoffe bewirkt und nachgewiesen werden kann.
- X.: Verfahren und Vorrichtung wie in den vorherigen Abschnitten definiert, die dadurch gekennzeichnet sind, dass die Kapillarplatte transparent oder opaque ist und einer optischen Messung frei zugänglich ist.
- Y.: Verfahren und Vorrichtung wie in den vorherigen Abschnitten definiert, die dadurch gekennzeichnet sind, dass lösliche oder suspendierbare Wirkstoffe fluoreszenzmarkiert sind und in die Kapillaren eingebracht werden.
- Z.: Verfahren und Vorrichtung wie in den vorherigen Abschnitten definiert, die dadurch gekennzeichnet sind, dass nach einer bestimmten Zeit die nichtgebunden Wirkstoffe entfernt werden und die gebundenen Wirkstoffe anhand der Fluoreszenzmarkierung nachgewiesen werden.
- AA.: Verfahren und Vorrichtung wie in den vorherigen Abschnitten definiert, die dadurch gekennzeichnet sind, dass die festphasen-gebundenen peptidischen oder peptoiden Zielmoleküle fluoreszenzmarkiert sind.
- BB.: Verfahren und Vorrichtung wie in den vorherigen Abschnitten definiert, die dadurch gekennzeichnet sind, dass lösliche oder suspendierbare Wirkstoffe, Säuren oder andere Flüssigkeiten in die Kapillaren gespült werden, nach einer bestimmten Zeit wieder ausgespült werden und das Vorhandensein der noch gebundenen fluoreszenzmarkierten Zielmoleküle nachgewiesen wird.
- CC.: Verfahren und Vorrichtung wie in den vorherigen Abschnitten definiert, die dadurch gekennzeichnet sind, dass die Fluoreszenzmarkierung mit Licht im UV, im Sichtbaren oder nahen Infrarot angeregt und detektiert wird.
- DD.: Verfahren und Vorrichtung wie in den vorherigen Abschnitten definiert, die dadurch gekennzeichnet sind, dass die Fluoreszenzanregung mit einem gepulsten Diodenlaser mit einer Pulsdauer von weniger als 10 ns geschieht.
- EE.: Verfahren und Vorrichtung wie in den vorherigen Abschnitten definiert, die dadurch gekennzeichnet sind, dass der Nachweis der Fluoreszenz zur Unterdrückung von Streulicht in einem Zeittor nach Abklingen des Anregungspulses geschieht.

## Patentansprüche

1. Verfahren zur Herstellung einer Substanzbibliothek, die zur Untersuchung molekularer Wechselwirkungen von löslichen und suspendierbaren Wirkstoffen mit festphasengebundenen peptidischen oder peptoiden Zielmolekülen dient, wobei die Substanzbibliothek eine zur Untersuchung notwendige Oberfläche innerhalb einer Platte (4) in Form einer Vielzahl von parallel angeordneten Kapillaren (1) beinhaltet, die Flüssigkeiten durch Kapillarkraft in die Kapillaren (1) ziehen und darin halten, umfassend die Schritte
a) Aufbringen einer Organosilanschicht, die funktionelle Gruppen zur Verankerung von peptidischen oder peptoiden Zielmolekülen aufweist, auf einer inneren Oberfläche der Kapillarplatte (4);
b) Anlegen von Probenarealen (3) bestehend aus einer Gruppe von benachbarten Kapillaren (1) durch jeweils ortsabhängiges Aufbringen einer die funktionelle Gruppe der Organosilanschicht temporär schützenden Substanz oder eines geschützten Ankermoleküls mit Hilfe eines Pipetierroboters;
c) Absättigung aller außerhalb der Probenareale (3) liegende Bereiche der Kapillarplatte (4) derart, dass weder eine Synthese oder Kopplung von Zielmolekülen noch eine unspezifische Bindung von Wirkstoffen erfolgen kann;
d) Entschützen der funktionellen Gruppen bzw. Ankermoleküle aller Probenareale (3) und
e) Kopplung oder Synthese peptidischer oder peptoider Zielmoleküle in den Probearealen (3), indem mit Hilfe eines Pipetierroboters Lösungen zur sequentiellen Synthese peptidischer oder peptoider Zielmoleküle aus Aminosäuren oder zur Anbindung vollständiger peptidischer oder peptoider Zielmoleküle in die entsprechenden Probenareale (3) der Kapillarplatte (4) pipettiert werden.

2. Verfahren nach Anspruch 1, **dadurch gekennzeichnet, dass** zur Silanisierung im Schritt a) γ-Aminopropyltrialkoxysilan oder andere organofunktionelle Silane verwendet werden.

3. Verfahren nach Anspruch 1 oder 2, **dadurch gekennzeichnet, dass** bei Verwendung von Metall- oder Halbmetallkappilarplatten vor der Silanisierung im Schritt a) eine Oxidschicht zur Bindung des Silans mittels Oberflächenoxidation der Kapillarplatte (4) geschaffen wird.

4. Verfahren nach Anspruch 1, **dadurch gekennzeichnet, dass** eine Vielzahl von Probenarealen (3) jeweils mit einem Durchmesser von bis zu 2.000 µm oder einer Anzahl von bis zu 4.000 benachbarten Kapillaren (1) auf einer Kapillarplatte (4) angelegt werden.

5. Verfahren nach Anspruch 1, **dadurch gekennzeichnet, dass** die Kapillaren (1) eine Länge sowie die Kapillarplatte (4) eine Dicke von 100 bis 2.000 µm besitzen.

6. Verfahren nach Anspruch 1, **dadurch gekennzeichnet, dass** im Schritt b) als geschütztes Ankermolekül Fluorenylmethoxycarbonyl(FMOC)-geschützter α-Amino-poly(ethylenglycol)-ω-propionsäure-N-hydroxybenzotriazolester eingesetzt wird.

7. Substanzbibliothek hergestellt gemäß dem Verfahren nach einem der Ansprüche 1 bis 6.

8. Substanzbibliothek nach Anspruch 7, **dadurch gekennzeichnet, dass** die Kapillarplatte (4) transparent oder opaque ist.

## Claims

1. Method for producing a substance library used for investigating molecular interactions of soluble and suspendable active substances with solid phase bound peptide or peptoid target molecules, wherein the substance library contains a surface, required for the investigation, within a plate (4) in the form of a large number of capillaries (1) which are positioned in parallel and which draw liquids into the capillaries (1) by capillary force and retain them therein, comprising the steps
a) application of an organosilane layer, which has functional groups for anchoring peptide or peptoid target molecules, to an inner surface of the capillary plate (4);
b) application of sample areas (3) consisting of a group of adjacent capillaries (1) by the respective position-dependent application of a substance which temporarily protects the functional group of the organosilane layer, or of a protected anchor molecule using a pipetting robot;
c) saturation of all the regions, outside the sample areas (3), of the capillary plate (4) such that it is impossible for synthesis or coupling of target molecules to take place or for an unspecific binding of active substances to occur;
d) deactivation of the functional groups or anchor molecules of all sample areas (3), and
e) coupling or synthesis of peptide or peptoid target molecules in the sample areas (3), by using a pipetting robot to pipette solutions for the sequential synthesis of peptide or peptoid target molecules from amino acids or to bind complete peptide or peptoid target molecules into the corresponding sample areas (3) of the capillary plate (4).

2. Method according to claim 1, **characterised in that** γ-aminopropyltrialkoxysilane or other organofunctional silanes are used for the silanisation process in step a).

3. Method according to either claim 1 or claim 2, **characterised in that** an oxide layer is produced to bind the silane by surface oxidation of the capillary plate (4) when using metal or semi-metal capillary plates before the silanisation process in step a).

4. Method according to claim 1, **characterised in that** a large number of sample areas (3), each having a diameter of up to 2,000 µm or having a number of up to 4,000 adjacent capillaries (1) are applied to a capillary plate (4).

5. Method according to claim 1, **characterised in that** the capillaries (1) have a length and the capillary plate (4) has a thickness of from 100 to 2,000 µm.

6. Method according to claim 1, **characterised in that** fluoroenylmethoxycarbonyl(FMOC)-protected α-amino-poly(ethyleneglycol)-ω-propionic acid-N-hydroxybenzotriazolester is used in step b) as the protected anchor molecule.

7. Substance library produced by the method according to any one of claims 1 to 6.

8. Substance library according to claim 7, **characterised in that** the capillary plate (4) is transparent or opaque.

## Revendications

1. Procédé pour produire une bibliothèque de substances qui sert à étudier les interactions moléculaires de principes actifs solubles et pouvant être mis en suspension avec des molécules cibles peptidiques ou peptoïdes liées à une phase solide, où la bibliothèque de substances contient une surface nécessaire à l'étude à l'intérieur d'une plaque (4) sous forme d'une multiplicité de capillaires (1) disposés parallèlement qui attirent les liquides par capillarité dans les capillaires (1) et qui les y maintiennent, comprenant les étapes
a) application d'une couche d'organosilane qui comporte des groupes fonctionnels pour l'ancrage de molécules cibles peptidiques ou peptoïdes, sur une surface interne de la plaque à capillaires (4) ;
b) disposition d'aires d'échantillons (3) consistant en un groupe de capillaires (1) voisins par application dépendante de site d'une substance protégeant temporairement le groupe fonctionnel de la couche d'organosilane ou d'une molécule d'ancrage protégée au moyen d'un robot de pipetage ;
c) saturation de tous les domaines de la plaque à capillaires (4) situés à l'extérieur des aires d'échantillons (3) de telle manière qu'il ne peut se produire ni une synthèse ou un couplage de molécules cibles ni une liaison non spécifique de principes actifs ;
d) déprotection des groupes fonctionnels ou des molécules d'ancrage de toutes les aires d'échantillons (3) et
e) couplage ou synthèse de molécules cibles peptidiques ou peptoïdes dans les aires d'échantillons (3) par le fait qu'à l'aide d'un robot de pipetage des solutions pour la synthèse séquentielle de molécules cibles peptidiques ou peptoïdes à partir d'aminoacides ou pour la liaison de molécules cibles peptidiques ou peptoïdes complètes sont pipetées dans les aires d'échantillons (3) correspondantes de la plaque à capillaires (4).

2. Procédé selon la revendication 1 **caractérisé en ce qu'**un γ-aminopropyltrialcoxysilane ou d'autres silanes organofonctionnels sont utilisés pour la silanisation.

3. Procédé selon la revendication 1 ou 2 **caractérisé en ce que**, dans le cas de l'utilisation de plaques à capillaires métalliques ou métalloïdiques, avant la silanisation dans l'étape a) une couche d'oxyde est créée pour la liaison du silane par oxydation superficielle de la plaque à capillaires (4).

4. Procédé selon la revendication 1 **caractérisé en ce qu'**une multiplicité d'aires d'échantillons (3) ayant chacune un diamètre pouvant atteindre 2000 µm ou un nombre pouvant atteindre 4000 capillaires (1) voisins sont disposées sur une plaque à capillaires (4).

5. Procédé selon la revendication 1 **caractérisé en ce que** les capillaires (1) possèdent une longueur et la plaque à capillaires (4) possède une épaisseur de 100 à 2000 µm.

6. Procédé selon la revendication 1 **caractérisé en ce que**, dans l'étape b), l'α-amino-poly(éthylèneglycol)-ω-propionate de N-hydroxybenzotriazole protégé par fluorénylméthoxycarbonyle (FMOC) est utilisé comme molécule d'ancrage protégée.

7. Bibliothèque de substances produite selon le procédé selon l'une des revendications 1 à 6.

8. Bibliothèque de substances selon la revendication 7 **caractérisée en ce que** la plaque à capillaires (4) est transparente ou opaque.
